Europäisches Patentamt

**European Patent Office**

**Office européen des brevets**

(19)

(11) Numéro de publication: **0 160 633**
**B1**

(12) # FASCICULE DE BREVET EUROPÉEN

(45) Date de publication du fascicule du brevet:
16.12.87

(51) Int. Cl.⁴: **A 61 F 5/47,** A 61 F 2/00

(21) Numéro de dépôt: **85870045.3**

(22) Date de dépôt: **22.03.85**

(54) Dispositif de fixation à la matrice, au cours de la période du post-partum immédiat, d'un dispositif anticonceptionnel intra-utérin.

(30) Priorité: **29.03.84 BE 212661**

(43) Date de publication de la demande:
**06.11.85 Bulletin 85/45**

(45) Mention de la délivrance du brevet:
**16.12.87 Bulletin 87/51**

(84) Etats contractants désignés:
**BE CH DE FR GB IT LI NL SE**

(56) Documents cité:
**EP-A-0 100 924**
**DE-A-2 505 941**
**DE-A-2 913 036**
**DE-A-2 946 833**
**FR-A-409 283**
**US-A-3 515 132**
**US-A-3 598 115**
**US-A-3 675 639**
**US-A-3 840 005**
**US-A-3 848 590**
**US-A-3 867 933**
**US-A-4 022 198**
**US-A-4 094 313**
**US-A-4 221 212**
**US-A-4 249 531**
**US-A-4 306 560**
**US-A-4 493 323**

(73) Titulaire: **Wildemeersch, Dirk, Vossenhul 8, B-8300 Knokke- Heist (BE)**

(72) Inventeur: **Wildemeersch, Dirk, Vossenhul 8, B-8300 Knokke- Heist (BE)**

(74) Mandataire: **Prignot, Jean, OFFICE HANSSENS SPRL Square Marie- Louise, 40 - bte 19, B-1040 Bruxelles (BE)**

LIBER, STOCKHOLM 1987

## Description

La présente invention a pour objet un dispositif d'insertion et de fixation à la matrice, au cours de la période du post-partum immédiat, d'un dispositif anticonceptionnel intra-utérin.

Depuis de nombreuses années, des études sont effectuées quant aux possibilités de placement d'un dispositif anticonceptionnel intra-utérin au cours de la période du post-partum immédiat, c'est-à-dire lors de l'eccouchement, immédiatement après la délivrance du placenta.

Cette méthode offre en effet de nombreux avantages, notamment dans les pays en voie de développement qui connaissent une surpopulation importante. Dans ces pays en effet, une grande proportion des femmes accouchent dans des hopitaux où le séjour est de courte durée. Dès lors, une méthode de placement d'un stérilet immédiatement après l'accouchement atteint une large population motivée, protège immédiatement contre une nouvelle grossesse, est indolore et n'interfère pas avec l'allaitement.

Malheureusement, aucun des dispositifs proposés à ce jour, consistant notamment en diverses formes de stérilets, n'apporte de solution satisfaisante. On constate en effet à la suite du placement de dispositifs anticonceptionnels intra-utérins au cours de la période du post-partum immédiat, un taux élévé d'expulsions variant, d'après les études effectuées, de 5 à 25 %, et un taux de translocation, empêchant un fonctionnement correct du stérilet et provoquant même des lésions à la matrice, plus élevé encore.

Il a déjà été proposé, notamment aux brevets US-A-3 598 115 et DE-A-2 505 941 des dispositifs pour introduire et fixer à la paroi du fond de la matrice, au cours de la période du post-partum immédiat, un stérilet qui est ainsi maintenu en place dans la matrice de manière sûre.

Le premier de ces documents, US-A-3 598 115, fait usage d'un dispositif conventionnel pour l'introduction d'un stérilet dans la matrice, et d'un stériliet particulier. Le stérilet comprend notamment un organe de fixation au tissu de la matrice, de rigidité suffisante pour pénétrer dans le tissu de la matrice sous l'action d'une poussée exercée sur l'arriere du sterilet. Une liaison aussi rigide entre le dispositif intra-utérin et son moyen de fixation à la matrice n'est pas souhaitable, et peut poser des problèmes lors du retour de la matrice à des dimensions normales, notamment si l'organe de fixation n'est pas solidarisé de la paroi du fond de la matrice de manière convenablement centrée.

Le second de ces documents, DE-A-2 505 941, révèle un dispositif d'insertion dans la matrice d'un dispositif anticonceptionnel intra-utérin solidaire d'un fil pourvu d'une pointe d'ancrage pour la fixation dans le tissu de la matrice. Ce dispositif comprend une enveloppe cylindrique entourant un piston creux pourvu à sa partie avant d'une aiguille sur laquelle se place la pointe d'ancrage. Le dispositif intra-utérin est placé entre le piston et l'enveloppe, le piston étant solidarisé temporairement, de manière libérable, de l'enveloppe, l'aiguille étant contenue dans l'enveloppe.

Le piston s'adapte à l'extrémité du doigt du chirurgien, et est ainsi introduit dans l'utérus. La désolidarisation du piston et de l'enveloppe est assurée par une pression de l'extrémité de l'enveloppe contre la paroi du fond de la matrice, cette désolidarisation étant suivie de la pénétration de l'aiguille dans le tissu de la matrice. L'aiguille est ensuite retirée et la pointe d'ancrage demeure dans la paroi de la matrice, le dispositif intra-utérin étant alors extrait du dispositif d'introduction par la traction du fil solidaire de la pointe d'ancrage.

Ce dispositif est peu aisé à utiliser, du fait qu'il nécessite l'introduction du doigt du chirurgien dans la matrice, et n'assure donc pas la vision de l'orientation du dispositif dans la matrice. En outre, aucune disposition autre que le serrage de l'élément d'ancrage sur l'aiguille n'est prise pour éviter une désolidarisation accidentelle de l'aiguille et du fil avant pénétration de l'aiguille dans le tissu de la matrice, ce qui enlèverait toute utilité à l'intervention. Enfin, c'est la traction sur l'élément d'ancrage prisonnier du tissu de la matrice qui permet le dégagement de l'aiguille et l'extraction du dispositif intra-utérin du dispositif d'introduction, une fois le fil mis en place dans le tissu de la matrice. Ceci peut provoquer l'arrachement de l'élément d'ancrage, et dès lors compromettre la solidarisation du dispositif intra-utérin à la matrice.

La présente invention a pour objet de remédier à ces inconvénients et de fournir un dispositif qui permette le placement d'un dispositif anticonceptionnel en position correcte dans la matrice, au cours de la période du post-partum immédiat, et son maintien sûr dans cette position au moins jusqu'au retour de la matrice à des dimensions normales. Rien ne s'oppose toutefois à ce que le stérilet soit maintenu dans la matrice au-delà de le période de retour de la matrice à des dimensions normales.

Ces buts sont atteints en prévoyant un dispositif d'insertion et de fixation à la matrice, au cours de la période du post-partum immédiat, d'un dispositif anticonceptionnel intra-utérin, ledit dispositif d'insertion comprenant:
- un fil, solidaire du dispositif anticonceptionnel et d'un élément d'accrochage,
- une aiguille pour l'insertion de l'élément d'accrochage solidaire du fil dans le tissu de la matrice,
- un élément de protection de l'aiguille,
- un élément de réception du dispositif anticonceptionnel,
- un élément d'actionnement de l'aiguille mobile par rapport à l'élément de protection,
- un moyen de blocage temporaire de l'élément d'actionnement par rapport à l'élément de protection dans lequel:
- le dispositif d'insertion se prolonge a son extrémité postérieure en un organe de préhension

- l'élément d'accrochage réalise un engagement sans serrage avec l'aiguille,
- des moyens sont prévus pour réaliser et maintenir une traction sur le fil, assurant la coopération de l'élément d'accrochage avec l'aiguille, aussi longtemps que les moyens de blocage de l'élément d'actionnement par rapport à l'élément de protection n'ont pas été dégagés et que le dispositif anticonceptionnel n'a pas été libéré de son élément de réception.

Suivant une autre caractéristique de l'invention,

- les moyens de blocage temporaire de l'élément d'actionnement par rapport à l'élément de protection sont des moyens commandés indépendamment de tout déplacement des éléments dont ils assurent le blocage,
- la commande des moyens de blocage est formée sur l'organe de préhension,
- des moyens de butée sont prévus pour limiter la pénétration de l'aiguille dans le tissu de la matrice.

Suivent une autre caractéristique de l'invention, la partie du fil destinée à être insérée dans la matrice comprend une déformation destinée à garantir la retenue du fil par le tissu de la matrice.

Suivant une caractéristique supplémentaire de l'invention, la déformation est un noeud formé dans le fil.

Suivant une autre caractéristique de l'invention, l'aiguille est une aiguille pourvue d'un tranchant et d'une fente destinée à recevoir une boucle du fil.

Suivant encore une autre caractéristique de l'invention, l'aiguille est une aiguille creuse pourvue d'un tranchant, et le fil présente un ergot destiné à s'insérer dans le canal de l'aiguille.

Suivant encore une autre caractéristique de l'invention, l'aiguille est une aiguille creuse à extrémité non tranchante, et le fil présente une déformation constituant d'un c°oté ergot, pour s'insérer dans le canal de l'aiguille, et de l'autre c°oté pointe, pour assurer la pénétration du fil poussé par l'aiguille dans le tissu de la matrice.

Suivant une autre caractéristique de l'invention, le fil et l'élément d'accrochage sont réalisés en une matière biologiquement neutre.

Suivant encore une autre caractéristique de l'invention, le fil et l'élément d'accrochage sont réalisés en une matière biodégradable.

Un autre but de l'invention est de fournir un dispositif qui comprend:

- une aiguille montée à l'extrémité avant d'
- une tige constituant élément d'actionnement, qui s'élargit à son extrémité arrière en un poucier,
- un élément de protection tubulaire, de dimensions suffisantes pour constituer également élément de maintien du dispositif anticonceptionnel,
- des moyens de blocage temporaire de l'élément de protection vis-à-vis de l'élément d'actionnement, dans une position dans laquelle l'extrémité avant de l'aiguille arrive au maximum à fleur de l'extrémité avant de l'élément de protection,

- des moyens de butée escamotables pour permettre d'abord un retrait limité de l'élément de protection, et ainsi autoriser une pénétration limitée de l'aiguille dans le tissu de la matrice, et pour permettre ensuite un retrait complémentaire de l'élément de protection, assurant la libération du dispositif intra-utérin, alors que le fil demeure maintenu dans le tissu de la matrice par le dispositif d'insertion.

Suivant une autre caractéristique de l'invention, le blocage temporaire de l'élément de protection vis-à-vis de l'élément d'actionnement est obtenu par solidarisation de ces éléments, à l'intervention d'une goupille traversant des alésages formés dans l'élément d'actionnement et dans l'élément de protection.

Suivant une autre caractéristique de l'invention, l'élément d'actionnement comporte un second alésage, traversé par une goupille constituant butée, à une distance de l'extrémité inférieure de l'élément de protection, lorsque ce dernier est solidarisé de l'élément d'actionnement, correspondant à la profondeur de pénétration souhaitée de l'aiguille dans le tissu de la matrice, et en ce que ce second alésage est lui même distant de la partie élargie de l'élément d'actionnement constituant poucier, d'une longueur correspondant au moins à la course de l'élément de protection nécessaire pour assurer la libération du dispositif intra-utérin.

Suivant une autre caractéristique de l'invention, l'élément de protection est un élément tubulaire, de longueur réduite, solidaire de bras se raccordant à un collier coulissant sur l'élément d'actionnement, et le blocage de l'élément tubulaire est obtenu à l'intervention d'ergots escamotables, formés dans l'élément d'actionnement et agissant sur le collier.

Un autre but de l'invention est de fournir un dispositif qui comprend une aiguille, un élément d'actionnement dont l'extrémité avant en forme de tiroir est destinée à recevoir un dispositif anticonceptionnel, et dont l'extrémité arrière s'élargit en forme de poucier, un élément de protection constituant glissière, dans lequel coulisse l'ensemble de l'aiguille et de l'élément d'actionnement, l'aiguille étant solidaire de l'extrémité avant en forme de tiroir de l'élément d'actionnement, et coopérant avec un fil solidaire du dispositif intra-utérin contenu dans l'extrémité avant en forme de tiroir de l'élément d'actionnement, des moyens étant prévus pour assurer une solidarisation temporaire de l'élément de protection et de l'élément d'actionnement, pour limiter la pénétration de l'aiguille dans le tissu de la matrice, ainsi que pour assurer l'expulsion du dispositif intra-utérin contenu dans l'extrémité avant en forme de tiroir de l'élément d'actionnement.

Suivant une autre caractéristique de l'invention, la longueur de l'aiguille est limitée à la profondeur de pénétration souhaitée de la dite aiguille dans le tissu de la matrice, et l'extrémité avant en forme de tiroir de l'élément d'actionnement constitue butée contre la paroi de la matrice pour limiter la profondeur de pénétration de l'aiguille dans le tissu de la matrice.

Suivant encore une autre caractéristique de l'invention, l'expulsion du dispositif intra-utérin est assurée par la présence d'une saillie, formée dans le fond de l'élément de protection à hauteur de la partie avant du tiroir, cette saillie coopérant avec le fil pour expulser le dispositif intra-utérin au cours de l'avancement de l'élément d'actionnement dans l'élément de protection, avancement qui provoque la pénétration de l'aiguille solidaire du fil dans le tissu de la matrice.

Suivant encore une autre caractéristique de l'invention, la solidarisation temporaire de l'élément de protection et de l'élément d'actionnement est assurée par l'engagement élastique d'un ergot formé sur l'élément d'actionnement, dans un logement formé sur l'élément de protection.

Encore un autre but de l'invention et de fournir un dispositif qui comprend un élément de protection tubulaire, pourvu à sa partie avant de deux rainures destinées à recevoir les bras d'un dispositif intra-utérin et a sa partie arrière d'une butée et d'une rainure, l'élément de protection se terminant en un manche, ainsi qu'un élément d'actionnement tubulaire, reçu dans l'élément de protection jusqu'au contact de la butée et solidaire à sa partie avant d'une aiguille et à sa partie arrière d'un poucier coopérant avec la rainure de l'élément de protection, le dispositif intra-utérin étant solidaire d'un fil pourvu d'un moyen d'accrochage au tissu de la matrice et coopérant avec l'aiguille, ainsi que d'un fil d'extraction constituant, par passage et traction au-travers des éléments d'actionnement et de protection, moyen de blocage du fil sur l'aiguille, du dispositif intra-utéarin dans les rainures, et de l'élément d'actionnement dans l'élément de protection.

Suivant une autre caractéristique de l'invention, la distance séparant le fond des rainures de la butée est au maximum égale à la distance longitudinale séparant les bras du dispositif anticonceptionnel, à l'état tendu du fil sur l'aiguille, de l'extrémité arrière de l'élément d'actionnement.

Suivant encore une autre caractéaristique de l'invention, le fond de la rainure constitue, par coopération avec le poucier butée limitant la pénétration de l'aiguille dans le tissu de la matrice.

Ces caractéristiques et d'autres seront mieux comprises en se reportant à la description, en même temps qu'au dessin annexé, qui représente, uniquement à titre d'exemple, divers modes de réalisation de l'invention et dans lequel:

- La fig. 1 représente schématiquement le placement, à l'aide d'un dispositif de l'invention, d'un dispositif anticonceptionnel dans la matrice au cours de la période du post-partum immédiat

- les fig. 2, 3 et 4 représentent diverses étapes du placement et de la fixation à la paroi du fond de la matrice du dispositif anticonceptionnel à l'aide d'un dispositif suivant l'invention,

- les fig. 5, 6 et 7 représentent divers modes de réalisation de dispositifs d'insertion du fil dans le tissu musculaire du fond de la matrice, et de fils associés à ces dispositifs,

- la fig. 8 donne une vue explosée des divers éléments d'un mode de réalisation d'un dispositif de fixation et de placement d'un dispositif intra-utérin suivant l'invention,

- la fig. 9 donne une vue en élévation d'un autre mode de réalisation d'un dispositif suivant l'invention,

- la fig. 10 est une vue en élévation, et en coupe suivant A-A à la fig. 11, d'un autre mode de réalisation d'un dispositif suivant l'invention,

- la fig. 11 montre, en plan, le dispositif de la fig. 10.

- les fig. 12 et 13 représentent en coupe, de dessus et de c°oté, encore un autre mode de réalisation, simplifié, d'un dispositif suivant l'invention.

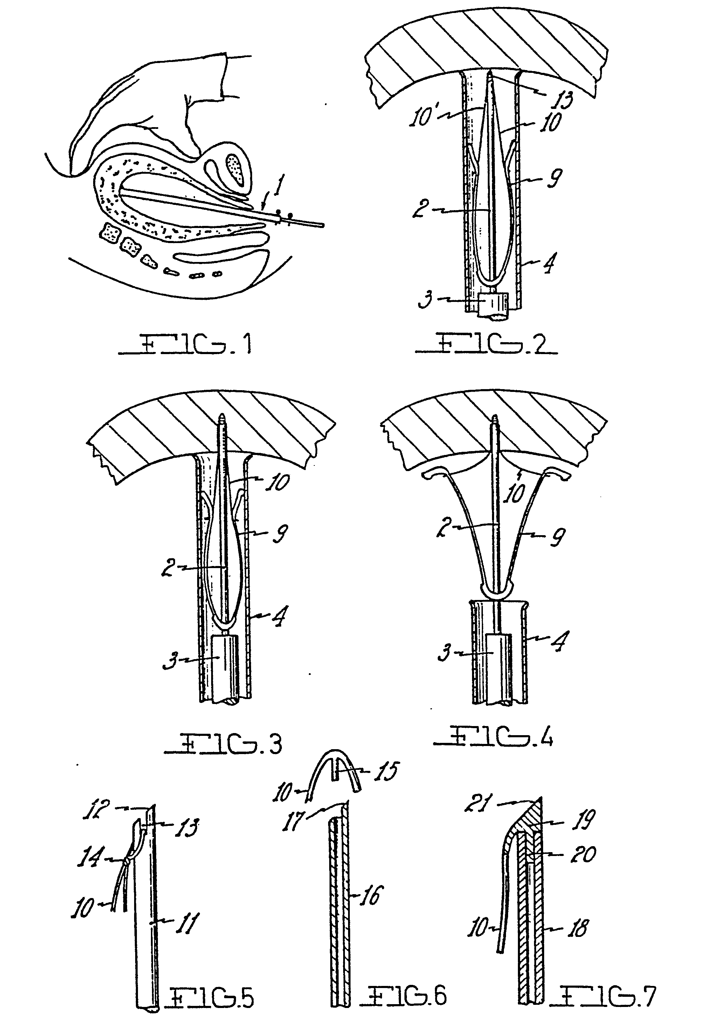

En se reportant au dessin, et plus particulièrement à la fig. 1 qui illustre à titre d'exemple un mode d'utilisation d'un dispositif d'insertion et de fixation à la matrice d'un dispositif anticonceptionnel suivant l'invention, immédiatement après la délivrance du placenta, une pression manuelle est exercée sur la matrice pour amener le col de la matrice aussi loin que possible vers l'avant du vagin, et un dispositif 1 suivant l'invention est introduit au-travers du col dilaté jusqu'à venir buter contre la paroi musculaire du fond de la matrice.

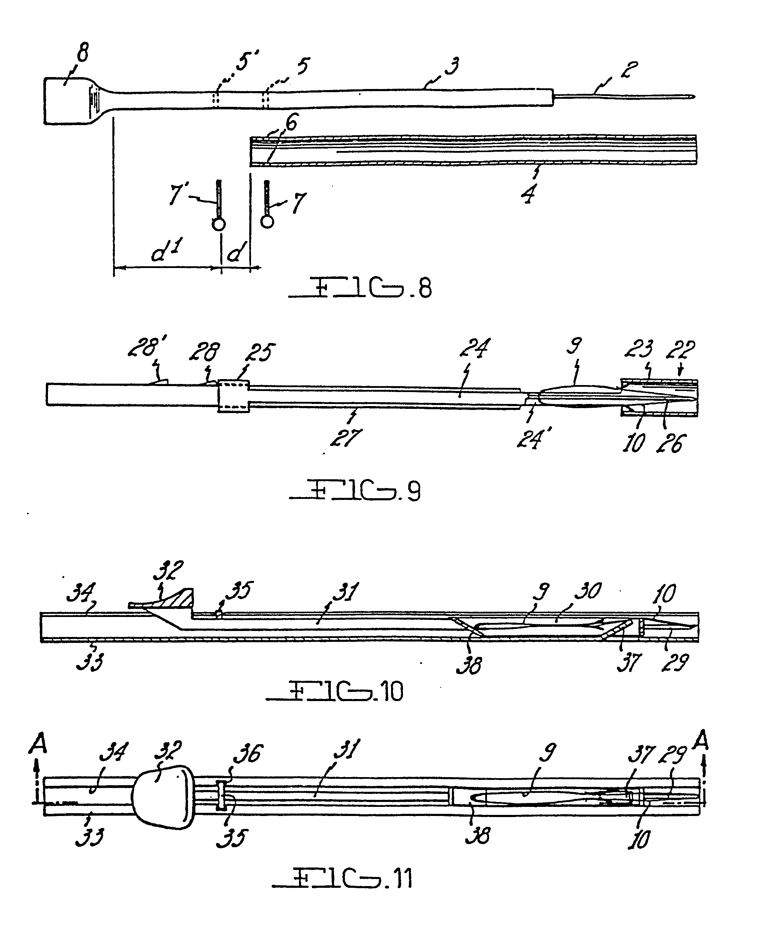

Le dispositif 1 illustré en fig. 1, qui constitue un mode de réalisation d'un dispositif suivant l'invention, est représenté plus en détail, quoique encore de manière schématique, et à l'exclusion du dispositif anticonceptionnel et du fil qui en est solidaire, en fig. 8.

Suivant ce mode de réalisation de la fig 8, le dispositif comprend essentiellement une aiguille 2 montée à l'extrémité avant d'une tige constituant élément d'actionnement 3, ainsi qu'un élément de protection tubulaire 4 (représenté en coupe). Des alésages 5, 5' sont percés dans l'élément d'actionnement 3 et des alésages diamétralement opposés sont formés dans la paroi de l'élément de protection tubulaire 4. Le dispositif comprend également des goupilles 7, 7' destinées a coopérer avec les alésages 5, 5' et 6 précités.

L'alésage 5 dans l'élément d'actionnement 3 et les alésages 6 dans l'élément de protection 4 sont agencés pour permettre, par coopération avec la goupille 7-lorsque les éléments d'insertion 2 et d'actionnement 3 sont introduits dans l'élément de protection 4 comme représenté partiellement et à plus grande échelle aux fig 2 à 4 - que l'élément de protection 4 soit solidarisé de l'élément d'actionnement 3. Dans cette position, l'extrémité avant de l'aiguille 2 affleure l'extrémité avant de l'élément de protection 4 comme représenté en fig 2.

L'alésage 5' est formé, dans l'élément d'actionnement 3, à un emplacement tel que la goupille 7', lorsqu'elle est introduite dans l'alésage 5', se trouve à une distance $d$ de l'extrémité arrière de l'élément de protection 4, solidarisé par la goupille 7 de l'élément d'actionnement 3, qui corresponde à la profondeur de pénétration souhaitée de l'aiguille 2 avec le fil dans le tissu de la matrice.

Suivant le mode de réalisation de la fig. 8, l'extrémité arrière de l'élément d'actionnement 3 est constituée sous forme d'un poucier 8.

L'alésage 5' est distant de l'élargissement du poucier 8 d'une distance $d^1$.

Le fonctionnement du dispositif de l'invention, appliqué à ce dispositif particulier, sera expliqué plus en détail en se reportant aux fig 2 à 4.

Lors de l'utilisation du dispositif de l'invention, on solidarise un dispositif anticonceptionnel intra-utérin tel que représenté d'accrochage au tissu de la matrice. Bien que le dispositif intra-utérin illustré au dessin soit un dispositif en V, il est évident que l'invention est utilisable avec tout autre type de dispositif anticonceptionnel intra-utérin. Le fil 10 est solidarisé de l'aiguille 2 et le dispositif intra-utérin 9 solidaire du fil 10 est ainsi introduit à l'état replié, par l'arrière, dans l'élément de protection 4 et avancé dans ce dernier.

L'élément de protection 4, sur la paroi interne duquel frotte le dispositif intra-utérin 9 replié, assure le maintien de celui-ci à l'état replié et la tension du fil 10 le reliant à l'aiguille 2. Cette tension maintient de manière sûre la solidarisation du fil 10 et de l'aiguille 2.

Lorsque les alésages 6 dans l'élément de protection 4 arrivent au niveau de l'alésage 5 dans l'élément d'actionnement 3, la goupille 7 est passée au-travers des alésages 6 et 5, solidarisant ainsi l'élément d'actionnement 3 à l'élément de protection 4. Comme dit plus haut, dans cette position l'avant de l'aiguille 2 affleure l'avant de l'élément de protection 4. Le dispositif est ainsi prêt à être introduit dans la matrice de la manière illustrée en fig. 1.

Le dispositif est introduit dans la matrice jusqu'à venir buter contre la paroi du fond de la matrice, comme représenté en fig. 1 et 2. Ainsi qu'on le voit plus clairement en fig. 2, la large extrémité de l'élément de protection 4 peut être amenée au contact de la paroi de la matrice sans risque de dommages pour cette dernière.

La goupille 7 est retirée alors que la goupille 7' est maintenue en position dans l'alésage 5'.

L'élément d'actionnement 3 est alors poussé en direction du fond de la matrice. L'élément de protection 4, qui n'est plus solidaire de l'élément d'actionnement 3, recule sur ledit élément d'actionnemant 3, tandis que l'aiguille 2, solidaire du fil 10, pénètre dans le tissu musculaire du fond de la matrice. Cette pénétration se poursuit jusqu'à ce que l'élément de protection 4 vienne buter contre la goupille 7'. L'élément de protection 4 a ainsi reculé d'une distance $d$ tandis que l'aiguille a pénétré dans le tissu de la matrice d'une distance correspondante et que le dispositif intra-utérin, entraîné par le fil 10, a avancé de la même distance dans l'élément de protection 4. Le dispositif se retrouve ainsi dans la position représentée en fig. 3.

La goupille 7' est alors *otée, et l'élément de protection 4 est retiré, sans qu'aucune poussée soit exercée en direction du fond de la matrice sur l'élément d'actionnement 3. Ce retrait est poursuivi sur la distance $d^1$ qui doit ainsi être au moins égale à la distance nécessaire pour assurer lé dégagement complet du dispositif intra-utérin 9 de l'élément de protection 4, comme représenté en fig. 4. Au cours du retrait de l'élément de protection 4, l'élément d'actionnement 3 est toutefois maintenu en position afin d'éviter que, suite à la friction des parois de l'élément 4 sur le dispositif anticonceptionnel, le fil 10 ne soit entraîné hors du tissu de la matrice.

Lorsque le retrait de l'élément de protection 4 est totalement accompli, l'ensemble du dispositif est retiré de la matrice, alors que le fil, solidaire du dispositif anticonceptionnel, est retenu par l'élément d'accrochage, dans le cas présent la boucle 10' du fil, par le tissu de la matrice.

Ainsi, le dispositif anticonceptionnel 9 demeure retenu par le fil 10 au tissu du fond de la matrice, et ne risque pas d'être expulsé. Afin d'éviter que le dispositif 1 se place mal dans la matrice, le fil 10 a une longueur telle qu'après insertion dans le tissu de la matrice, le dispositif anticonceptionnel ne puisse s'écarter de manière substantielle de la position dans laquelle il a été introduit dans la matrice.

Le jeu dans le fil est toutefois suffisant pour permettre au dispositif intra-utérin, si le fil n'a pas été inséré exactement dans la partie médiane de la paroi du fond de la matrice, de se placer correctement dans la matrice revenue à des dimensions normales, sans que des tensions importantes se manifestent dans le fil fixé à la matrice.

Dans le mode de réalisation représenté aux fig. 2 à 4, le dispositif d'insertion 2 comprend une aiguille 11 munie d'un tranchant 12 et d'une fente axiale 13, comme représenté à grande échelle à la fig. 5. Toutefois, dans ce mode de réalisation des fig. 2 à 4, le fil 10 constitue une simple boucle 10' par solidarisation aux deux branches du dispositif anticonceptionnel, et cette boucle constituant élément d'accrochage au tissu de la matrice est passée dans la fente 13 de l'aiguille 11.

On peut aussi prévoir de réaliser dans le fil une déformation constituant élément d'accrochage au tissu de la matrice, afin d'améliorer la retenue du fil par le tissu de la matrice. C'est ainsi qu'une des solutions les plus simples consiste à former un noeud 14 dans le fil, et à utiliser pour l'insertion du fil dans le tissu de la matrice une aiguille fendue telle que celle illustrée en fig. 5.

Une autre solution, utilisable avec des fils monofilamentaires, consiste à former un ergot 15 dans le fil et à utiliser une aiguille creuse 16 pourvue d'un tranchant 17. L'ergot 15, introduit sans serrage dans le canal de l'aiguille 16, assure la retenue du fil 10 sur l'aiguille sous l'effet de la tension du fil, alors que le dégagement du fil de l'aiguille s'effectue sans aucun effort sur le fil, par simple retrait de l'aiguille. Cette solution est représentée à la fig. 6 où, pour la facilité de l'illustration, l'aiguille est représentée de profil et le fil de face.

Une autre solution encore consiste à utiliser une aiguille creuse 18 dépourvue de tranchant et à former dans le fil une déformation 19 constituant d'un côté ergot 20 pour la solidarisation sans serrage à l'aiguille et de l'autre côté pointe 21 pour la pénétration du tissu de la matrice sous la poussée de l'aiguille 18. Après retrait de l'aiguille, la déformation 19 dégagée de l'aiguille par simple coulissement de l'ergot 20, contribue à la retenue du fil dans le tissu de la matrice.

Ces diverses solutions sont bien entendu données à titre d'exemple, et d'autres modes de réalisation d'une solidarisation sans serrage du fil à l'aiguille apparaîtront immédiatement à l'homme de métier, qui tous entrent dans le cadre de la présente invention.

Un autre mide de réalisation de l'invention est représenté en fig. 9.

Suivant ce mode de réalisation, le dispositif de protection 22 et de maintien du dispositif anticonceptionnel est réalisé sous forme d'un court élément tubulaire 23 qui se prolonge en deux bras 24, 24', qui eux-mêmes se raccordent à un collier 25. Le dispositif comporte également une aiguille 26 et un élément d'actionnement 27 solidaire de l'aiguille 26. L'élément d'actionnement 27 comporte deux butées élastiquement escamotables 28, 28' écartés l'une de l'autre d'une distance correspondant à la distance d à la fig. 8.

Le fonctionnement de ce dispositif est substantiellement le même que celui du dispositif de la fig. 8 décrit ci-dessus. Toutefois, comme le collier 25 vient seulement au contact de la butée 28, sans solidarisation réelle à l'élément d'actionnement 27, on prendra garde, lors de la manipulation du dispositif, que le collier 25 ne soit jamais avancé sur l'élément d'actionnement 27. En effet, un tel mouvement vers l'avant risquerait de provoquer le désengagement du fil 10 et de l'aiguille 26 avant que ce dernier ait été introduit dans le tissu de la matrice.

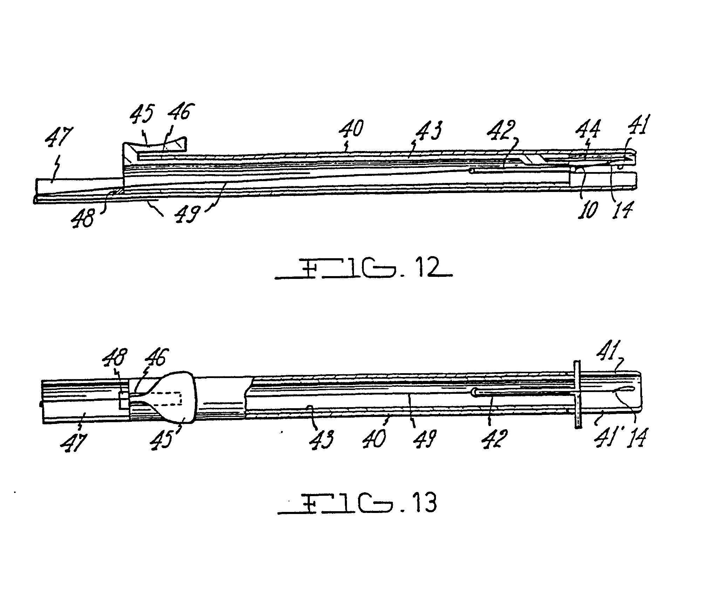

Les fig. 10 et 11 montrent, respectivement en élévation et en coupe, et en plan, un autre mode de réalisation d'un dispositif selon l'invention.

Suivant ce mode de réalisation, le dispositif comprend une aiguille 29 solidaire de l'extrémité avant 30 en forme de tiroir, d'un élément d'actionnement 31 qui se termine, à son autre extrémité, par un poucier 32.

L'élément d'actionnement 31 coulisse dans un guide creux 33, pourvu d'une fente longitudinale 34, constituant élément de protection.

L'élément d'actionnement 31 est solidaire d'un ergot 35 susceptible de coopérer avec un logement 36 formé de part et d'autre de la fente 34.

Le fond de l'élément de protection 33 présente une déformation 37 constituant plan incliné, se situant à l'avant du tiroir lorsque l'ergot 35 coopère avec le logement 36. De même, l'arrière du tiroir constitue paroi inclinée 38.

En fonctionnement, le dispositif intra-utérin 9 est coincé entre les parois latérales du tiroir 30, dont l'écartement correspond substantiellement à la largeur de la fente 34 et le fil 10 est solidarisé à l'état tendu de l'aiguille 29. L'ergot 35 coopère avec le logement 36 et l'extrémité avant de l'aiguille arrive au maximum à fleur de l'extrémité avant de l'élément de protection 33.

Le dispositif, dans cette position, est amené au contact de la paroi du fond de la matrice.

Ensuite, par une pression vers le bas sur le poucier 32, suivie d'une poussée vers l'avant, on dégage d'abord l'ergot 35 du logement 36, et on provoque ensuite le déplacement de l'élément d'actionnement 31 et de l'aiguille 29 dans l'élément de protection 33.

Sous l'effet de ce déplacement, l'élément d'insertion 29 fait saillie de l'élément de protection et pénétre dans le tissu de la matrice, y entraînant le fil 10.

Simultanément, le dispositif anticonceptionnel 9 est entraîné par le tiroir 30 jusqu'à venir au contact du plan incliné 37. Etant donné que le maintien du dispositif anticonceptionnel 9 est assuré principalement à la partie avant du tiroir, et en fonction de la tension et de l'élasticité plus ou moins grande du fil, le plan incliné 37, tout en faisant remonter le dispositif anticonceptionnel 9, aura tendance à le faire également reculer dans le tiroir 30. Pour éviter un coincement de la partie arrière du stérilet dans le tiroir, la paroi arrière 38 du tiroir est inclinée vers l'arrière. La coopération du fil 10, empêchant le dispositif anticonceptionnel de reculer substantiellenent dans le tiroir, du plan incliné fixe 37 et de l'avance du tiroir 30 obligera ledit dispositif anticonceptionnel à monter le long du plan incliné 37 jusqu'à être expulsé par la fente 34.

La libération du dispositif anticonceptionnel peut s'effectuer sans risque avant que le fil n'ait été complètement inséré dans le tissu de la matrice. En effet, dès le début de la pénétration de l'aiguille avec le fil dans le tissu de la matrice, la solidarisation du fil à l'aiguille est maintenue par le tissu de la matrice.

Suivant ce mode de réalisation, l'aiguille 29 a une longueur correspondant à la profondeur de pénétration souhaitée dans le tissu de la matrice, et l'avant du tiroir 30 constitue donc butée pour limiter la pénétration de l'élément 29 dans le tissu de la matrice.

En se reportant au mode de réalisation des fig. 12 et 13, le dispositif comprend un élément de protection tubulaire 40 pourvu à l'avant de rainures 41, 41' pour recevoir les bras d'un dispositif intra-utérin 42, ainsi qu'un élément d'actionnement 43, également de forme générale tubulaire, solidaire à son extrémité avant d'une aiguille 44 coopérant avec un fil 10 solidaire du dispositif intra-utérin 42.

L'élément d'actionnement 43 est solidaire à sa partie arrière d'un poucier 45 s'engageant dans une rainure longitudinale 46 formée dans l'élément de protection 40.

L'élément de protection 40 se raccorde à sa partie arrière à un manche 47 constituant, avec l'extrémité arrière de l'élément de protection, un organe de préhension.

A l'arrière de l'élément de protection 40 est formée une butée 48 destinée à coopérer avec la partie arrière de l'élément d'actionnement 43.

Le dispositif intra-utérin 42 est solidaire à sa partie arrière d'un fil 49 constituant fil d'extraction lorsque le dispositif intra-utérin est en place dans la matrice. A titre d'exemple, le stérilet 42 est un stérilet en "T".

Dans ce mode de réalisation, c'est le fil 49, passant au-travers de l'élément de protection 40 et de l'élément d'actionnement 43 et sortant à l'arrière de l'élément de protection 40 qui constitue moyen de blocage de l'élément d'actionnement 43.

A cet effet, la distance séparant l'extrémité des rainures 41, 41' de la butée 48 est au maximum égale à la distance longitudinale séparant les bras du dispositif à l'état tendu du fil 10 sur l'aiguille 44 de l'extrémité arrière de l'élément d'actionnement 43. De la sorte, une traction vers l'arrière sur le fil 49 assure, par l'intermédiaire du dispositif intra-utérin 42, le blocage de l'élément d'actionnement 43 contre la butée 48 dans l'élément de protection 40. De même, cette traction assure un maintien fiable du dispositif intra-utérin dans l'élément de protection 40, ainsi que du fil 10 sur l'aiguille 44.

Cette traction dans le fil est assurée, une fois le fil 49 tendu, par pliage et maintien de l'extrémité du fil 49 à l'extérieur de l'élément de protection 40 ou du manche 47.

En utlisation, le dispositif est ainsi introduit dans la matrice jusqu'a ce que l'extrémité avant de l'élément de protection 40, qui est arrondie, vienne au contact de la paroi du fond de la matrice. L'on exerce alors une poussée vers l'avant sur le poucier 45 en lâchant le fil 49. Ceci provoque l'avancement de l'élément d'actionnement 43, ainsi que la pénétration de l'aiguille 44, solidaire du fil 10, dans le tissu de la matrice.

La libération du fil 49 assure également la libération du dispositif intra-utérin 42, dont les bras peuvent se déplacer sans frottement substantiel dans les rainures 41, 41'. L'on peut ainsi de manière sûre retirer de la matrice le dispositif d'introduction, sans risquer de désolidariser le stérilet de la paroi de la matrice.

Suivant un mode de réalisation préféré de l'invention, l'extrémité de la rainure 46 constitue butée pour limiter l'avancement de l'élément d'actionnement 43, et donc la pénétration de l'aiguille dans la paroi de la matrice.

L'invention a été décrite et illustrée à simple titre d'exemple nullement limitatif, et il va de soi que de nombreuses modifications peuvent être apportées à sa réalisation sans s'écarter de son objet tel qu'indiqué dans les revendications ci-jointes.

**Revendications**

1. Dispositif d'insertion et de fixation à la matrice, au cours de la période du post-partum immédiat, d'un dispositif anticonceptionnel intra-utérin, lesdit dispositif d'insertion comprenant:
- un fil (10), solidaire du dispositif anticonceptionnel (9; 40) et d'un élément d'accrochage (10'; 14; 15; 19),
- un aiguille (2; 26; 29; 44), pour l'introduction de l'élément d'accrochage (10'; 14; 15; 19), solidaire du fil dans le tissu de la matrice,
- un élément de protection (4; 22; 33; 40) de l'aiguille,
- un élément de réception (4; 22; 30; 41, 41') du dispositif anticonceptionnel,
- un élément d'actionnement (3; 27; 31; 43) de l'aiguille, mobile par rapport à l'élément de protection,
- un moyen de blocage temporaire (5, 6, 7; 25, 28; 35, 36; 48, 49) de l'élément d'actionnement par rapport à l'élément de protection,
ledit dispositif d'insertion étant caractérisé en ce que:
- le dispositif d'insertion se prolonge à son extrémité postérieure en un organe de préhension (8; 25, 27; 32, 33; 47, 40),
- l'élément d'accrochage (10'; 14; 15; 19) réalise un engagement sans serrage avec l'aiguille (2; 26; 29; 44)
- des moyens (4, 22; 30; 49) sont prévus pour réaliser et maintenir une traction sur le fil (10), assurant la coopération de l'élément d'accrochage (10'; 14; 15; 19) avec l'aiguille (2; 26; 29; 44) aussi longtemps que les moyens de blocage (5, 6, 7; 25, 28; 35, 36; 48, 49) de l'élément d'actionnement par rapport à l'élément de protection n'ont pas été dégagés et que le dispositif anticonceptionnel n'a pas été libéré de son élément de réception (4; 22; 30; 41, 41').

2. Dispositif suivant la revendication 1, caractérisé en ce que:
- les moyens de blocage (5, 6, 7; 25, 28; 35, 36; 48, 49) de l'élément d'actionnement par rapport à l'élément de protection, sont des moyens commandés indépendamment de tout déplacement des éléments (3, 4; 22, 27; 31, 33; 40, 43) dont ils assurent le blocage,
- la commande des moyens de blocage est formée sur l'élément de préhension,
- des moyens de butée (7'; 28'; 30; 46) sont prévus pour limiter la pénétration de l'aiguille dans le tissu de la matrice.

3. Dispositif suivant les revendications 1 et 2, caractérisé en ce que la partie du fil destinée à être insérée dans la matrice comprend une déformation (14; 15; 19) destinée à garantir la ratenue du fil par le tissu de la matrice.

4. Dispositif suivant la revendication 3, caractérisé en ce que la déformation est un noeud (14) formé dans le fil.

5. Dispositif suivant l'une quelconque des revendications 2, 3 ou 4, caractérisé en ce que l'aiguille (2; 26; 29) est une aiguille (11) pourvue d'un tranchant (12) et d'une fente (13) destinée à recevoir une boucle du fil (10).

6. Dispositif suivant la revendication 2, caractérisé en ce que l'aiguille (2; 26; 29) est une aiguille creuse (16) pourvue d'un tranchant (17), et en ce que le fil (10) présente un ergot (15) destiné à s'insérer dans le canal de l'aiguille.

7. Dispositif suivant la revendication 2, caractérisé en ce que l'aiguille (2; 26; 29) est une aiguille creuse (18) à extrémité non tranchante, et en ce que le fil (10) présente une déformation (19) constituant d'un c*oté ergot (20), pour s'insérer dans le canal de l'aiguille, et de l'autre c*oté pointe (21), pour assurer la pénétration du fil poussé par l'aiguille dans le tissu de la matrice.

8. Dispositif suivant l'une quelconque des revendications 2 à 7, caractérisé en ce que le fil (10) et l'élément d'accrochage (10'; 14; 15; 19) sont réalisés en une matière biologiquement neutre.

9. Dispositif suivant l'une quelconque des revendications 2 à 7, caractérisé en ce que le fil (10) et l'élément d'accrochage (10'; 14; 15; 19) sont réalisés en une matière biodégradable.

10. Dispositif suivant l'une quelconque des revendications 2 à 9, caractérisé en ce que -l'aiguille (2; 26) est montée à l'extrémité avant d'une tige constituant élément d'actionnement (3; 27), ladite tige s'élargissant à son extrémité arrière en un poucier (8),
- l'élément de protection (4; 22) est tubulaire et de dimensions suffisantes pour constituer également élément de maintien du dispositif anticonceptionnel (9),
- les moyens de blocage (5, 6, 7; 28) temporaire de l'élément de protection (4; 22) vis-à-vis de l'élément d'actionnement (3; 27) sont dans une position dans laquelle l'extrémité avant de l'aiguille (2; 26) arrive au maximum à fleur de l'extrémité avant de l'élément de protection (4; 22),
- des moyens de butée (5', 7'; 28') escamotables sont prévus pour permettre d'abord un effacement limité de l'élément de protection, et ainsi autoriser une pénétration limitée de l'aiguille dans le tissu de la matrice, et pour permettre ensuite un retrait complémentaire de l'élément de protection, assurant la libération du dispositif intra-utérin, alors que le fil demeure maintenu dans le tissu de la matrice par le dispositif d'insertion.

11. Dispositif suivant la revendication 10, caractérisé en ce que le blocage temporaire de l'élément de protection (4) vis-à-vis de l'élément d'actionnement est obtenue par solidarisation de ces éléments à l'intervention d'une goupille (7) traversant des alésages (5, 6) formés dans l'élément d'actionnement (3) et dans l'élément de protection (4).

12. Dispositif suivant la revendication 11, caractérisé en ce que l'élément d'actionnement (3) comporte un second alésage (5'), traversé par une goupille (7'), à une distance de l'extrémité inférieure de l'élément de protection, lorsque ce dernier est solidarisé de l'élément d'actionnement, correspondant à la profondeur de pénétration souhaitée de l'aiguille dans le tissu de la matrice, et en ce que ce second alésage est lui même distant de la partie élargie de l'élément d'actionnement constituant poucier, d'une longueur correspondant au moins à la course de l'élément de protection nécessaire pour assurer la libération du dispositif intra-utérin.

13. Dispositif suivant la revendication 10, caractérisé en ce que l'élément de protection (22) est un élément tubulaire (23), de longueur réduite, solidaire de bras (24, 24') se raccordant à un collier (25) coulissant sur l'élément d'actionnement (27), et en ce que le blocage de l'élément de protection (22) est obtenu à l'intervention d'ergots escamotables (28, 28'), formés dans l'élément d'actionnement (27) et agissant sur le collier (25).

14. Dispositif suivant l'une quelconque des revendications 2 à 9, caractérisé en ce que l'élément d'actionnement (31) comporte l'extrémité avant en forme de tiroir (30) destinée à recevoir un dispositif anticonceptionnel (9) et l'extrémité arrière qui s'élargit en un poucier (32), et en ce que l'élément de protection (33) constitue une glissière, dans laquelle coulisse l'ensemble de l'aiguille (29) et de l'élément d'actionnement (31), l'aiguille (29) étant solidaire de l'extrémité avant (30) en forme de tiroir de l'élément d'actionnement (31), et coopérant avec un fil (10) solidaire du dispositif intra-utérin (9) contenu dans l'extrémité avant en forme de tiroir (30) de l'élément d'actionnement (31), des moyens étant prévus pour assurer une solidarisation temporaire de l'élément de protection et de l'élément d'actionnement, pour limiter la pénétratiom de l'aiguille dans le tissu de la matrice, ainsi que pour assurer l'expulsion du dispositif intra-utérin contenu dans l'extrémité avant en forme de tiroir de l'élément d'actionnement.

15. Dispositif suivant la revendication 14, caractérisé en ce que la longueur de l'aiguille (29) est limitée à la profondeur de pénétration souhaitée de la dite aiguille dans le tissu de la matrice, et en ce que l'extrémité avant en forme de tiroir (30) de l'élément d'actionnement constitue butée contre la paroi de la matrice pour limiter la profondeur de pénétration de l'aiguille (29) dans le tissu de la matrice.

16. Dispositif suivant les revendications 14 et 15, caractérisé en ce que l'expulsion du dispositif intra-utérin est assurée à l'intervention d'une saillie (37), formée dans le fond de l'élément de protection à hauteur de la partie avant du tiroir, cette saillie (37) coopérant avec le fil pour expulser le dispositif intra-utérin (9) au cours de l'avancement de l'élément d'actionnement (31) dans l'élément de protection (33), avancement qui provoque la pénétration de l'aiguille (29) solidaire du fil (10) dans le tissu de la matrice.

17. Dispositif suivant les revendications 14 à 16, caractérisé en ce que la solidarisation temporaire de l'élément de protection (33) et de l'élément d'actionnement (31) est assurée par l'engagement élastique d'un ergot (35) formé sur l'élément d'actionnement (31), dans un logement (36) formé sur l'élément de protection (33).

18. Dispositif suivant l'une quelconque des revendications 1, 3 à 9, caractérisé en ce que l'élément de protection (40) est un élément tubulaire, pourvu à sa partie avant de deux rainures (41, 41') destinées à recevoir les bras lateraux d'un dispositif intra-utérin (42) et à sa partie arrière d'une butée (48) et d'une rainure (46), l'élément de protection (40) se terminant en un manche (47), et en ce que l'élément d'actionnemant (43) est également un élément tubulaire, reçu dans l'élément de protection (40) jusqu'au contact de la butée et solidaire à sa partie avant d'un aiguille (44) et à sa partie arrière d'un poucier (45) coopérant avec la rainure (46) de l'élément de protection (40), le dispositif intra-utérin (42) étant solidaire d'un fil (10) pourvu d'un moyen d'accrochage au tissu de la matrice et coopérant avec l'aiguille (44), ainsi que d'un fil d'extraction (49) constituant, par passage et traction au-travers des éléments d'actionnement (43) et de protection (40), moyen de blocage du fil (10) sur l'aiguille (44), du dispositif intra-utérin (42) dans les rainures (41, 41'), et de l'élément d'actionnement (43) dans l'élément de protection (40).

19. Dispositif suivant la revendication 18, caractérisé en ce que la distance séparant le fond des rainures (41, 41') de la butée (48) est au maximum égale à la distance longitudinale séparant les bras lateraux du dispositif anticonceptionnel, à l'état tendu du fil (10) sur l'aiguille (44), de l'extrémité arrière de l'élément d'actionnement (43).

20. Dispositif suivant l'une quelconque des revendications 18 ou 19, caractérisé en ce que le fond de la rainure (46) constitue, par coopération avec le poucier (45) butée limitant la pénétration de l'aiguille (44) dans le tissu de la matrice.

## Claims

1. A device for the insertion and the fixation of an intrauterine contraceptive device to the uterine fundus of a female in the immediate post-partum period, such device comprising:
- a thread (10) affixed to the contraceptive device (9; 40) and to a retaining member (10'; 14; 15; 19),
- a needle (2; 26; 29; 44) for the insertion of the retaining member (10'; 14; 15; 19) attached to the thread into the uterine muscle,
- a protecting member (4; 22; 33; 40) for the needle,
- a receiving member (4; 22; 30; 41, 41') for the contraceptive device,
- an actuating member (3; 27; 31; 43) for the needle, movable with respect to the protecting member,
- means (5, 6, 7; 25, 28; 35, 36; 48, 49) for temporarily locking the actuating member with regard to the protecting member, said device for the insertion being characterized in that,
- the device for the insertion extends backwards to form a gripping member (8; 25, 27; 32, 33; 47, 40),
- the retaining member (10'; 14; 15; 19) loosely engages the needle (2; 26; 29; 44),
- means (4; 22; 30; 49) are provided for achieving and maintaining a traction on the thread (10), ensuring the cooperation of the retaining member (10'; 14; 15; 19) with the needle (2; 26; 29; 44) as long as the means (5, 6, 7; 25, 28; 35, 36; 48, 49) for locking the actuating member with regard to the protecting member are not disengaged and the contraceptive device is not released from its receiving member (4; 22; 30; 41, 41').

2. A device according to claim 1, characterized in that:
- the means (5, 6, 7; 25, 28; 35, 36; 48, 49) for locking the actuating member with respect to the protecting member are means which are controlled independently of any displacement of the members (3, 4; 22, 27; 31, 33; 40, 43) which they lock,
- the control mechanism of the locking means is provided on the gripping member,
- stop means (7'; 28'; 30; 46) are provided for limiting the penetration depth of the needle into the uterine muscle.

3. A device according to claims 7 and 2, characterized in that the portion of the thread to be inserted into the uterine wall is provided with a deformation (14; 15; 19) whereby the thread is retained by the uterine muscle.

4. A device according to claim 3, characterized in that the deformation is a knot (14) in the thread.

5. A device according to anyone of claims 2, 3 or 4, characterized in that the needle (2; 26; 29) is a needle (11) provided with a sharp edge (12) and a slot (13) for receiving a loop of the thread (10).

6. A device according to claim 2, characterized in that the needle (2; 26; 29) is a hollow needle (16) provided with a sharp edge (17), and the thread (10) is provided with a pin (15) to be inserted in the lumen of the needle.

7. A device according to claim 2, characterized in that the needle (2; 26; 29) is a hollow needle (18) without sharp edge at forwards end, and the thread (10) is provided with a deformation (19) comprising at one end a pin (20) to be inserted in the canal of the needle, and at the other end a point (21), to ensure the penetration of the thread, pushed by the needle, into the uterine muscle.

8. A device according to anyone of claims 2 to 7, characterized in that the thread (10) and the retaining member (10'; 14; 15; 19) are made of a biologically inert material.

9. A device according to anyone of claims 2 to 7, characterized in that the thread (10) and the retaining member (10'; 14; 15; 19) are made of a bio-degradable material.

10. A device according to anyone of claims 2 to 9, characterized in that:
- the needle (2; 26) is provided at the front end of a stem which serves as actuating member (3; 27), said stem enlarging at its rear end in a thumb-piece (8),
- the protecting member (4; 22) is tubular and has dimensions sufficient for making also holding member for the contraceptive device (9),
- the means (5, 6, 7; 28) for temporarily locking the protecting member (4; 22) with respect to the actuating member (3; 27) is in a position in which the front end of the needle (2; 26) is at most flush with the front end of the protecting member (4; 22),
- retractable stop means (5', 7'; 28') are providedd allowing in a first step a limited backwar movement of the protecting member with regard to the needle, and thus a limited penetration of the needle into the uterine muscle, then further backward withdrawal of the protecting member, releasing the contraceptive device while the thread is kept maintained in the uterine muscle by the needle.

11. A device according to claim 10, characterized in that temporary locking of the protecting member (4) with regard to the actuating member is obtained by locking these members by a pin (7) extending through bores (5, 6) in the actuating member (3) and the protecting member (4).

12. A device according to claim 11, characterized in that the actuating member (3) is provided with a second bore (5'), receiving a pin (7') which, when the protecting member is interlocked with the actuating member, is located at a distance from the rear end of the protecting member which corresponds with the desired penetration depth of the needle in the uterine muscle, and in that said second bore is located at a distance from the enlarged part of the actuating member, which makes the thumb-piece, which corresponds at least to the travel of the protecting member necessary for releasing the contraceptive device.

13. A device according to claim 10, characterized in that the protecting member (22) is a tubular member (23) of restricted length, integral with two legs (24, 24') connected to a collar (25) sliding on the actuating member (27), and in that the locking of the protecting member (22) is obtained through retactable pins (28, 28') provided in the actuating member (27), acting on the collar (25).

14. A device according to anyone of claims 2 to 9, characterized in that the actuating member (31) has a drawer-like front part (30) which is intended to receive the contraceptive device, and a rear part which enlarges to form a thumb-piece (32), and in that the protecting member (33) makes a guide, in which slides the actuating member (31) with the needle (29), the needle (29) being secured to the drawer-like front part (30) of the actuating member (31) and cooperating with a thread (10) secured to the contraceptive device (9) which is kept in the drawer-like front part (30) of the actuating member (31), means being provided for ensuring the temporary locking of the protecting member with respect to the actuating member, for limiting the penetration depth of the needle in the uterine muscle, and also for ensuring expulsion of the contraceptive device kept in the drawer-like front part of the actuating member.

15. A device according to claim 14, characterized in that the length of the needle (29) is limited to the desired penetration depth of the needle in the uterine muscle, and in that the drawer-like front part (30) of the actuating member makes a stop unit which limits the penetration depth of the needle (29) into the uterine muscle, by abutting against the uterine fundus.

16. A device according to claims 14 and 15, characterized in that the expulsion of the contraceptive device is obtained through a flange (37), provided in the bottom of the protecting member at the front part of the drawer-like member, whereby this flange (37) cooperates with the thread in order to expel the contraceptive device (9) while the actuating member (31) is moved forward with respect to the protecting member (33), which movement causes the penetration of the needle (29) with the thread (10) into the uterine muscle.

17. A device according to claims 14 to 16, characterized in that the temporary interlocking of the protecting member (33) and the actuating member (31) is ensured by the resilient engagement of a detent (35) on the actuating member (31) into a recess (36) in the protecting member (33).

18. A device according to anyone of claims 1, 3 to 9, characterized in that the protecting member (40) is a tubular member provided with two slots (41, 41') at its front end, for receiving the side legs of a contraceptive device (42), and with a stop (48) and a slot (46) at its rear end, the protecting member (40) ending in a handle (47), and in that the actuating member (43) is also a tubular member, received in the protecting member (40) in abutment with the stop, the tubular actuating member (43) being secured at its front end to a needle (44) and at its rear end to a thumb-piece (45) cooperating with the slot (46) in the protecting member (40), the contraceptive device being affixed to a thread (10) provided with a retaining member cooperating with the needle (44), and to an extraction thread (49) which makes, by passage and traction through the actuating (43) and protecting (40) members, means for securing the thread (10) to the needle (44) and means for locking the contraceptive device (42) in the slots (41, 41') and the actuating member (43) in the protecting member (40).

19. A device acccording to claim 18, characterized in that the distance between the bottom of the slots (41, 41') and the stop (48) is at most equal to the longitudinal distance between the side legs of the contraceptive device, when the thread (10) is tightly engaged with the needle (44), and the rear end of the actuating member (43).

20. A device according to anyone of claims 18 or 19, characterized in that the bottom of the slot (46) makes, through cooperation with the thumb-piece (45), a stop limiting the penetration of the needle (44) in the uterine muscle.

**Patentansprüche**

1. Vorrichtung zum Einsetzen und Befestigen eines intrauterinen Empfängnisverhütungsmittels in die bzw. an der Gebärmutter im sofortigen Postpartum, umfassend:
- einen Faden (10), der mit dem Empfängnisverhütungsmittel (9; 40) und einem Verankerungselement (10'; 14; 15; 19) verbunden ist,
- eine Nadel (2; 26; 29; 44) zum Einführen des mit dem Faden verbundenen Verankerungselementes (10'; 14; 15; 19) in das Gewebe der Gebärmutter,
- ein Schutzelement (4; 22; 33; 40) für die Nadel,
- ein Aufnahmeelement (4; 22; 30; 41, 41') für das Empfängnisverhütungsmittel,
- ein Betätigungselement (3; 27; 31; 43) für die Nadel, das gegenüber dem Schutzelement beweglich ist,
- ein Blockierungsmittel (5, 6, 7; 25, 28; 35, 36; 48, 49) zum zeitweiligen Blockieren des Betätigungselementes relativ zum Schutzelement,
wobei die Einsetzvorrichtung dadurch gekennzeichnet ist, daß
- sie an ihrem hinteren Ende zu einem Grifforgan (8; 25, 27; 32, 33; 47, 40) verlängert ist,
- das Verankerungselement (10'; 14; 15; 19) mit der Nadel (2; 26; 29; 44) klemmfrei in Eingriff steht,
- Mittel (4; 22; 30; 49) vorgesehen sind, um einen Zug auf den Faden (10) auszuüben und aufrechtzuerhalten, der das Zusammenwirken des Verankerungselementes (10'; 14; 15; 19) mit der Nadel (2; 26; 29; 44) solange gewährleistet, wie die Blockierungsmittel (5, 6, 7; 25, 28; 35, 36; 48, 49) zum Blockieren des Betätigungselementes relativ zum Schutzelement nicht gelöst wurden und das Empfängnisverhütungsmittel nicht aus seinem Aufnahmelement (4; 22; 30; 41, 41') freigegeben wurde.

2. Vorrichtung nach Anspruch 1, dadurch gekennzeichnet, daß
- die Blockierungsmittel (5, 6, 7; 25, 28; 35, 36; 48, 49) zum Blockieren des Betätigungselementes relativ zum Schutzelement unabhängig von jeder Verrückung der Elemente (3, 4; 22, 27; 31, 33; 40, 43) steuerbar sind, deren Blockierung sie bewirken,
- die Steuerung der Blockierungsmittel an dem Griffelement ausgebildet ist,
- Anschlagmittel (7'; 28'; 30; 46) vorgesehen sind, um das Eindringen der Nadel in das Gewebe der Gebärmutter zu begrenzen.

3. Vorrichtung nach den Ansprüchen 1 und 2, dadurch gekennzeichnet, daß der zum Einführen in die Gebärmutter bestimmte Abschnitt des Fadens eine Verformung (14; 15; 19) aufweist, die dazu bestimmt ist, das Festhalten des Fadens durch das Gewebe der Gebärmutter zu gewährleisten.

4. Vorrichtung nach Anspruch 3, dadurch gekennzeichnet, daß die Verformung von einem Knoten (14) im Faden gebildet ist.

5. Vorrichtung nach einem der Ansprüche 2, 3 oder 4, dadurch gekennzeichnet, daß die Nadel (2; 26; 29) eine mit einer Schneide (12) und einem Schlitz (13) versehene Nadel (11) ist, wobei letztererzur Aufnahme einer Schleife des Fadens (10) bestimmt ist.

6. Vorrichtung nach Anspruch 2, dadurch gekennzeichnet, daß die Nadel (2; 26; 28) als Hohlnadel (16) ausgebildet ist, die eine Schneide (17) aufweist, und daß der Faden (10) einen Zapfen (15) aufweist, der zum Einstecken in den Kanal der Nadel bestimmt ist.

7. Vorrichtung nach Anspruch 2, dadurch gekennzeichnet, daß die Nadel (2; 26; 29) eine Hohlnadel (18) mit einem nichtschneidenden Ende ist und daß der Faden (10) eine Verformung (19) aufweist, die auf der einen Seite einen zum Einführen in den Nadelkanal bestimmten Zapfen (20) und auf der anderen Seite eine Spitze (21) bildet, der das Eindringen des von der Nadel gestoßenen Fadens in das Gewebe der Gebärmutter gewährleistet.

8. Vorrichtung nach einem der Ansprüche 2 bis 7, dadurch gekennzeichnet, daß der Faden (10) und das Verankerungselement (10'; 14; 15; 19) aus einem biologisch neutralen Material hergestellt sind.

9. Vorrichtung nach einem der Ansprüche 2 bis 7, dadurch gekennzeichnet, daß der Faden (10) und das Verankerungselemente (10'; 14; 15; 19) aus einem biologisch abbaubaren Material hergestellt sind.

10. Vorrichtung nach einem der Ansprüche 2 bis 9, dadurch gekennzeichnet, daß
- die Nadel (2; 26) am vorderen Ende eines Stabes angeordnet ist, der das Betätigungselement (3; 27) bildet und sich an seinem rückwärtigen Ende zu einem Drücker (8) verbreitert,
- das Schutzelement (4; 22) rohrförmig ist und ausreichende Abmessungen aufweist, um gleichzeitig ein Halteelement für das Empfängnisverhütungsmittel (9) zu bilden,
- die Blockierungsmittel (5, 6, 7; 28) zum zeitweiligen Blockieren des Schutzelementes (4; 22) relativ zum Betätigungselement (3; 27) eine Position einnehmen, in der das vordere Ende der Nadel (2; 26) höchstens mit dem vorderen Ende des Schutzelementes (4; 22) fluchtet,
- ausrückbare Anschlagmittel (5', 7'; 28') vorgesehen sind, um zunächst ein begrenztes Zurückziehen des Schutzelementes und damit ein begrenztes Eindringen der Nadel in das Gewebe der Gebärmutter zu ermöglichen und um anschließend ein weiteres Zurückziehen des Schutzelementes zu erlauben, das die Freigabe des intrauterinen Empfängnisverhütungsmittels gewährleistet, während der Faden durch die Einsetzvorrichtung im Gewebe der Gebärmutter festgehalten bleibt.

11. Vorrichtung nach Anspruch 10, dadurch gekennzeichnet, daß die zeitweilige Blockierung des Schutzelementes (4) relativ zum Betätigungselement durch eine Verbindung dieser Elemente mittels eines Splintes (7) erzielt wird, der Bohrungen (5; 6) in dem Betätigungselement (3) und dem Schutzelement (4) durchsetzt.

12. Vorrichtung nach Anspruch 11, dadurch gekennzeichnet, daß das Betätigungselement (3) eine von einem Splint (7') durchsetzte zweite Bohrung (5') aufweist, die einen Abstand vom unteren Ende des Schutzelementes besitzt, wenn letzteres mit dem Betätigungselement verbunden ist, wobei der Abstand der gewünschten Eindringtiefe der Nadel in das Gewebe der Gebärmutter entspricht, und daß die zweite Bohrung selbst von dem den Drücker bildenden verbreiterten Abschnitt des Betätigungselementes einen Abstand aufweist, dessen Länge mindestens der Bewegungsstrecke des Schutzelementes entspricht, die erforderlich ist, um die Freigabe des intrauterinen Empfängnisverhütungsmittels zu gewährleisten.

13. Vorrichtung nach Anspruch 10, dadurch gekennzeichnet, daß das Schutzelement (22) ein Rohrelement (23) reduzierter Länge ist, das mit Armen (24, 24') verbunden ist, die ihrerseits mit einer auf dem Betätigungselement (27) gleitend verschiebbaren Hülse (25) verbunden sind, und daß die Blockierung des Schutzelementes (22) mittels ausrückbarer Nasen (28, 28') erzielt wird, die an dem Betätigungselement (27) angeordnet sind und an der Hülse (25) angreifen.

14. Vorrichtung nach einem der Ansprüche 2 bis 9, dadurch gekennzeichnet, daß das vordere Ende des Betätigungselementes (31) in Form einer Schublade (30) ausgebildet ist, die zur Aufnahme des Empfängnisverhütungsmittels (9) bestimmt ist, wogegen sich das hintere Ende des Betätigungselementes zu einem Drücker (32) erweitert, und daß das Schutzelement (33) eine Gleitführung bildet, in der die die Nadel (29) und das Betätigungselement (31) umfassende Einheit gleitet, wobei die Nadel (29) mit dem in Schubladenform ausgebildeten vorderen Ende (30) des Betätigungselementes (31) verbunden ist und mit einem Faden (10) zusammenwirkt, der seinerseits mit dem in dem schubladenförmigen vorderen Ende (30) des Betätigungselementes (31) enthaltenen intrauterinen Empfängnisverhütungsmittel (9) verbunden ist, und wobei Mittel vorgesehen sind, um eine zeitweilige Verbindung des Schutzelementes und des Betätigungselementes zu gewährleisten und um das Eindringen der Nadel in das Gewebe der Gebärmutter zu begrenzen sowie das Ausstoßen des in dem schubladenförmigen vorderen Ende des Betätigungselementes enthaltenen intrauterinen Empfängnisverhütungsmittels sicherzustellen.

15. Vorrichtung nach Anspruch 14, dadurch gekennzeichnet, daß die Länge der Nadel (29) auf die gewünschte Eindringtiefe der Nadel in das Gewebe der Gebärmutter begrenzt ist und daß das schubladenförmige vordere Ende (30) des Betätigungselementes einen zur Anlage an der Gebärmutterwand bestimmten Anschlag bildet, um die Eindringtiefe der Nadel (29) in das Gebärmuttergewebe zu begrenzen.

16. Vorrichtung nach den Ansprüchen 14 und 15, dadurch gekennzeichnet, daß das Ausstoßen des intrauterinen Empfängnisverhütungsmittels durch Hilfe eines Vorsprunges (37) sichergestellt ist, der am Boden des Schutzelementes auf Höhe des vorderen Teils der Schublade ausgebildet ist und der mit dem Faden zusammenwirkt, um das intrauterine Empfängnisverhütungsmittel (9) während des Vorschiebens des Betätigungselementes (31) in dem Schutzelement (33) auszustoßen, wobei das Vorschieben das Eindringen der mit dem Faden (10) verbundenen Nadel (29) in das Gewebe der Gebärmutter bewirkt.

17. Vorrichtung nach den Ansprüchen 14 bis 16, dadurch gekennzeichnet, daß die zeitweilige Verbindung des Schutzelementes (33) und des Betätigungselementes (31) durch elastischen Eingriff einer an dem Betätigungselement (31) ausgebildeten Nase (35) mit einer Aussparung (36) an dem Schutzelement (33) gewährleistet ist.

18. Vorrichtung nach einem der Ansprüche 1, 3 bis 9, dadurch gekennzeichnet, daß das Schutzelement (40) ein rohrförmiges Element ist, das an seinem vorderen Ende mit zwei Nuten (41, 41') versehen ist, die zur Aufnahme der seitlichen Arme eines intrauterinen Empfängnisverhütungsmittels (42) bestimmt sind, und das an seinem hinteren Ende einen Anschlag (48) und eine Nut (46) aufweist, wobei das Schutzelement (40) in einer Hülse (47) endet, und daß das Betätigungselement (43) ebenfalls als rohrförmiges Element ausgebildet ist, das in dem Schutzelement (40) bis zum Kontakt mit dem Anschlag eingeführt ist und an seinem vorderen Abschnitt mit einer Nadel (44) und an seinem hinteren Abschnitt mit einem Drücker verbunden ist, der mit der Nut (46) in dem Schutzelement (40) zusammenwirkt, wobei das intrauterine Empfängnisverhütungsmittel mit einem Faden (10) verbunden ist, der mit einem Mittel zur Verankerung an dem Gebärmuttergewebe versehen ist und mit der Nadel (44) zusammenwirkt, und ferner mit einem Ausziehfaden (49) verbunden ist, der aufgrund des Durchtritts und des Zuges durch das Betätigungselement (43) und das Schutzelement (40) Mittel zur Blockierung des Fadens (10) an der Nadel (44), zur Blockierung des intrauterinen Empfängnisverhütungsmittels in den Nuten (41, 41') und zur Blockierung des Betätigungselementes (43) in dem Schutzelement (40) bildet.

19. Vorrichtung nach Anspruch 18, dadurch gekennzeichnet, daß der Abstand, der den Grund der Nuten (41, 41') von dem Anschlag (48) trennt, höchstens gleich dem Längsabstand ist, der die beiden seitlichen Arme des Empfängnisverhütungsmittels bei Spannung des Fadens (10) an der Nadel (44) von dem rückwärtigen Ende des Betätigungselementes (43) trennt.

20. Vorrichtung nach einem der Ansprüche 18 oder 19, dadurch gekennzeichnet , daß der Grund der Nut (46) durch Zusammenwirken mit dem Drücker (45) einen Anschlag bildet, der das Eindringen der Nadel (44) in das Gewebe der Gebärmutter begrenzt.

0 160 633

FIG.1

FIG.2

FIG.3

FIG.4

FIG.5

FIG.6

FIG.7

FIG.8

FIG.9

FIG.10

FIG.11

y

FIG. 12

FIG. 13